(19) Europäisches Patentamt
European Patent Office
Office européen des brevets

(11) Publication number: **0 600 342 A1**

(12) **EUROPEAN PATENT APPLICATION**

(21) Application number: **93118790.0**

(22) Date of filing: **23.11.93**

(51) Int. Cl.[5]: **C07D 307/00**, C12P 17/04, A61K 31/34

(30) Priority: **04.12.92 EP 92120686**

(43) Date of publication of application: **08.06.94 Bulletin 94/23**

(84) Designated Contracting States: **AT BE CH DE DK ES FR GB GR IE IT LI LU MC NL PT SE**

(71) Applicant: **F. HOFFMANN-LA ROCHE AG**
**Postfach 3255**
**CH-4002 Basel(CH)**

(72) Inventor: **Ohtsuka, Tatsuo**
**615 Kamimachiya**
**Kamakura-shi, Kanagawa-ken(JP)**
Inventor: **Sakai, Akiko**
**1970-1-612 Kosugaya-cho,**
**Sakae-ku,**
**Yokohama-shi**
**Kanagawa-ken(JP)**
Inventor: **Okuda, Toru**
**1-6-11, Kugenuma Tachibana,**
**Fujisawa-shi**
**Kanagawa-ken(JP)**

(74) Representative: **Grossner, Lutz, Dr. et al**
**Grenzacher Strasse 124**
**Postfach 3255**
**CH-4002 Basel (CH)**

(54) **Hispidospermidin and pharmaceutical compositions.**

(57) The compound represented by the formula,

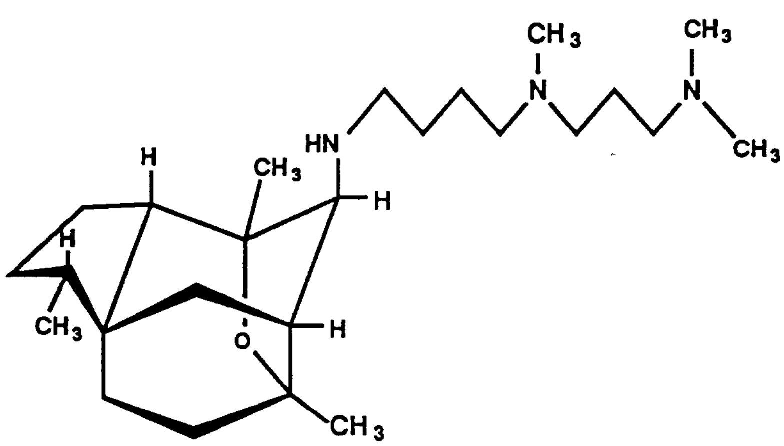

is obtained from cultures of *Chaetosphaeronema hispidulum.* The compound is an inhibitor of phospholipase C.

EP 0 600 342 A1

The present invention is concerned with a novel compound (hereinafter referred to as hispidospermidin) represented by the formula I,

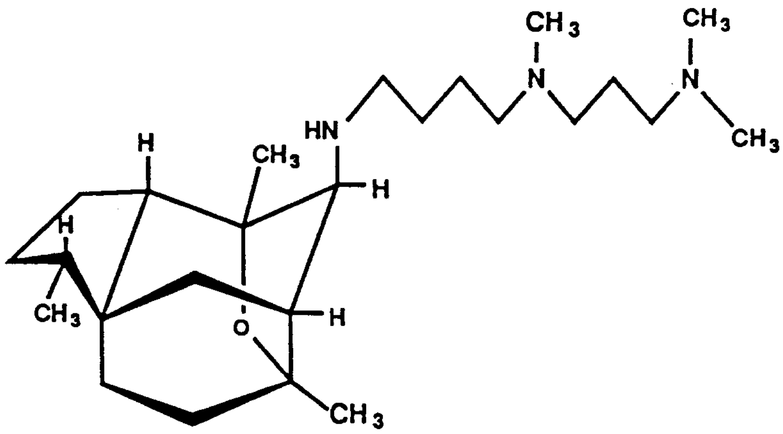

I

The present invention is also concerned with a pharmaceutical composition containing hispidospermidin as phospholipase C (PLC) inhibiting composition, and a process for producing hispidospermidin.

It is well known that inositolphosphates and their degraded products play an important role in the signal transduction for cell growth and transformation. One of these signaling pathways is proceeded by activation of PLC. The binding of first messengers such as mitogens, hormones, growth factors, neurotransmitters to their specific cellular receptors, activates the enzyme, PLC. Activated PLC cleaves phosphatydylinositol 4,5-diphosphate ($PIP_2$) into 2 kinds of second messengers, namely diacylglycerol (DG) and inositol triphosphate ($IP_3$). DG activates protein kinase C (PKC) and $IP_3$ increases intracellular $Ca^{++}$ level. These two second messengers trigger various physiological responses of cells through different pathways.

From these facts it follows that PLC is one of the effective targets to block deregulated proliferative and/or inflammatory signals. So PLC inhibiting activity is predictive of anti-tumor and/or anti-inflammatory activity.

In accordance with the present invention is has been found that hispidospermidin has potent PLC inhibiting activity.

The physico-chemical properties of hispidospermidin as described in the Example given hereinbelow as follows:

Appearance: Colorless oil
$[a]_D^{24}$ -60° (*c* 1.45, $CHCl_3$)
Molecular formula: $C_{25}H_{47}N_3O$
*HREI-MS (*m/z*) $M^+$: Calcd. for $C_{25}H_{47}N_3O$: 405.3720
Found.: 405.3730

$$UV\ l^{MeOH}_{max}\ nm:$$

End absorption
IR $n_{max}$ (neat) $cm^{-1}$: 3600~3100,2780,2760
Solubility: Soluble in MeOH, $CHCl_3$, acetone, acidic water
Insoluble in water
$^1H$ NMR (400 $MH_z$, $CDCl_3$) d:
(TMS was used as an internal standard.)

0.82 (3H, d, J=7 Hz), 1.12 (3H, s), 1.13(1H, m), 1.22 (3H, s), 1.22 (1H, dd, J=8,13 Hz), 1.28 (1H, d, J=12.5 Hz), 1.40 (1H), 1.42 (1H, m), 1.45 (1H, dd, J=5, 12.5 Hz), 1.48 (1H), 1.50 (2H, m), 1.51 (2H, m), 1.52 (1H, m), 1.57 (1H, m), 1.64 (2H, m), 1.71 (1H, m), 1.73 (1H, m), 1.77 (1H, dd, J=7.5, 13 Hz), 2.03 (1H, t, J=5 Hz), 2.22 (9H, s), 2.28 (2H, br t, J=7Hz), 2.34 (2H), 2.35 (2H), 2.60 (2H, t, J=7 Hz), 2.81 (1H, d,

* HREI-MS: High resolution electron impact mass spectrometry.

J = 5 Hz)
$^{13}C$ NMR (100 $MH_z$, $CDCl_3$) d:
(TMS was used as an internal standard.)

14.1, 18.1, 20.3, 21.0, 25.1, 25.6, 27.3, 28.6, 28.9, 29.4, 32.2, 42.3, 43.2,43.4 44.5, 45.6, 48.6, 51.9, 55.9, 57.8, 58.0, 66.4, 80.0, 81.7

According to the process provided by the present invention, hispidespermidin is produced by cultivating a microorganism belonging to the genus *Chaetosphaeronema* capable of producing hispidospermidin under aerobic condition in an aqueous culture medium and isolating hispidospermidin from the culture.

The microorganism used in the foregoing process can be any strain (including variants) belonging to the genus *Chaetosphaeronema* capable of producing hispidospermidin. Especially preferred strains are *Chaetosphaeronema hispidulum* NR7127 as well as variants thereof. *Chaetosphaeronema hispidulum* NR7127 was isolated from a soil sample and identified as a strain belonging to the genus *Chaetosphaeronema*.

The strain denoted as *Chaetosphaeronema hispidulum* NR7127 has been deposited with the Fermentation Research Institute, Agency of Industrial Science and Technology, Japan, under the Budapest Treaty on

November 25, 1992 as follows:

*Chaetosphaeronema hispidulum* (CdA) Moesz NR 7127 (FERM BP-4081)

The culture characteristics and the morphological characteristics of *Chaetosphaeronema hispidulum* NR7127 (FERM-BP 4081) are as follows:

Cultural characteristics

Dark brown to black colonies grew on malt extract agar showing floccose appearance. Exudates or soluble pigments were not produced. No conidiogenesis was observed under normal fluorescent or natural light. Under near UV light, however, numerous conidiomata half submerged in agar were formed after several weeks. Cultivation on banana leaf agar was the most effective for conidiomata production.

Morphological characteristics

The conidiomata were pycnidial, dark brown to black, globose to subglobose, and up to 450 µm in diam. Ostioles were single, central and slightly beaked. Numerous bristly setae were restricted around the beak and were septate and straight. The conidiogenous cells with collarette were phialidic, cylindrical and hyaline, 6.0-10.5 x 3.0-7.5 µm, and their periclinal walls were thick. The conidia were hyaline, one-septate, smooth, straight to slightly curved, and their apex and base were obtuse. Their size was 11.5 - 16.0 x 1.5 - 3.5 µm.

The conidiomata had numerous setae. The conidiogenous cells were phialidic. The conidia were one-septate, hyaline and straight to curved. There characteristics clearly indicated that this strain was included in the genus *Chaetosphaeronema* Moesz (Sutton, 1980). Further properties such as the sizes of conidiomata, conidiogenous cells and conidia indicated that this strain should be *Chaetosphaeronema hispidulum* Moesz. The beaks of this strain were not as long as those of the herbarium specimen, *Chaetosphaeronema hispidulum* IMI 182342. In a personal communication with Dr. Sutton (International Mycological Institute,Kew, England), he suggested that the difference can be neglected. Therefore this strain was identified as *Chaetosphaeronema hispidulum* Moesz.

The cultivation in accordance with the process provided by the present invention can be carried out in a culture medium which contains customary nutrients usable by the microorganism being cultivated. As carbon sources there can be mentioned, for example, glucose, sucrose, starch, glycerol, molasses, dextrin and mixtures thereof. Nitrogen sources are, for example, soybean meal, cottonseed meal, meat extract, peptone, dried yeast, yeast extract, cornsteep liquor, ammonium sulfate, sodium nitrate and mixtures thereof. Moreover, there may be added to the culture medium other organic or inorganic substances for promoting the growth of the microorganism and for increasing the production of hispidospermidin, examples of such substances being inorganic salts such as, for example, calcium carbonate, sodium chloride, phosphates and the like.

The cultivation is carried out under aerobic conditions in an aqueous medium, preferably by submerged fermentation. The cultivation is suitably carried out at a temperature of 20°C - 35°C, the optimal temperature being 27°C. The cultivation is preferably carried out at a pH of 3 to 9. The cultivation time depends on the conditions under which the cultivation is carried out. In general, it is sufficient to carry out the cultivation for 50 ~ 200 hours.

The isolation of hispidospermidin from the fermentation broth can be carried out according to methods known per se. For example, the mycelium can be separated from the fermentation broth by centrifugation or filtration and hispidospermidin can be extracted from the filtrate with a water-immiscible organic solvent such as alkanol e.g. n-butanol and esters e.g. ethyl acetate, butyl acetate etc. On the other hand, hispidospermidin contained in the separated mycelium can be obtained, for example, by extracting the mycelium with a solvent such as aqueous acetone or aqueous methanol, removing the solvent and further extracting the residue with a water-immiscible organic solvent. The thus-obtained solvent layer is dried over a dehydrating agent such as sodium sulfate etc. and then concentrated under reduced pressure. The resulting crude hispidospermidin can be purified by means of extraction methods, partition methods, precipitation methods, column-chromatographical methods (using silica gel, aluminium oxide, Diaion HP-21, ion exchange resin etc. as adsorbents).

Inhibitory activity of hispidospermidin against PLC was measured.

The assay mixture (0.1 ml) contained 200 mM Tris-acetate buffer (pH 5.5), 2 mM $CaCl_2$, 250 μM PI (suspended in the buffer, specific activity: 800 dpm/nmole) and enzyme.

In the standard assay, 20 μl of inhibitor in 15 mM Tis-HCl buffer (pH 7.5) was added to the substrate suspension (50 μl). The reaction was started with addition of the enzyme solution prepared from partially purified rat brain PLC (30 μl, ca. 5 μg protein) and incubated for 20 min. at 30 °C. The reaction was terminated by adding 0.3 ml of unlabeled PI (200 μM) solution containing 1 mg/ml of bovine serum albumin and 1 mM EGTA, followed by adding of 0.3 ml of 7.5% ice cold trichloroacetic acid containing 3 mM $CaCl_2$. Under the conditions, the enzyme hydrolyzed about 30 % of substrate in the assay mixture. As a positive control showing 100% inhibition of PLC, 20 μl of 25 mM EDTA was added to the assay mixture.

The incubation mixture was kept on ice for 10 min asn centrifuged at 1,700 x g for 10 min. The radioactivity of an aliquot (0.2 ml) of the supernatant was measured by liquid scintillation counter (ALOKA).

Inhibitory activity of hispidospermidin against PLC is shown in Table I.

Table I

| Compounds | $IC_{50}$ (μM) |
|---|---|
| Hispidospermidin | 15.7 |
| Quinacrine | 314.4 |
| Tobramycin | 29.9 |
| Sperimine | 59.3 |
| Trifluoperazine | 149.7 |

Acute toxicity of hispidospermidin is not observed.

The novel hispidospermidin provided by the present invention can find use as medicaments particularly in the treatment of inflammatory diseases, such as psoriasis and allergy, and cancer, such as breast cancer, colorectal cancer and lung cancer. The medicaments can be particularly for the treatment of tumors and/or inflammatory conditions. The medicaments can be for oral or parenteral application, in the form of unit dose pharmaceutical preparations which contain them or their salts in admixture with an organic or inorganic inert carrier material suitable for enteral application, such as for example water, gelatine, gum arabic, lactose, starch, magnesium stearate, talc, vegetable oils, polyalkylene glycols etc. The unit dose pharmaceutical preparations can be present in solid form, e.g. as tablets, coated tablets, dragees or capsules, hard gelatine or sort gelatine, or in liquid form, e.g. as solutions, syrups, or suspensions.

A dose unit may contain 10 to 200 mg of active ingredient. The daily dosage for an adult can be in the range from 10 to 400 mg and may be varied according to individual requirements which can be determined by those of ordinary skill in the art.

The following example further illustrates the present invention.

Example

A portion of the stock culture (0.1 ml) of *Chaetosphaeronema hispidulum* NR7127 (FERM-BP No. 4081) was inoculated into a 500 ml flask containing 100 ml of a medium consisting of 2% glucose, 2% potato starch, 2% Toast soya, 0.5% yeast extract, 0.25% NaCl, 0.005% $ZnSO_4 \cdot 7H_2O$, 0.0005% $CuSO_4 \cdot 5H_2O$, 0.0005% $MnSO_4 \cdot 4H_2O$, 0.32% $CaCO_3$ and 0.03% Nissan disfoam CA-115. The pH of the medium was adjusted to 7.0 before the addition of calcium carbonate. This seed culture was shaken on a rotary shaker at 190 rpm at 27 °C for 4 days. Two ml of the resultant culture was transferred into each of six

500 ml flasks containing the same medium and cultured under the same conditions for 3 more days. Six hundred milliliters of the second seed culture was inoculated into a 50-liter jar fermentor containing 30 liters of the same medium and 0.3% disfoam. The fermentation was conducted at 27 °C at an aeration rate of 30 liters/min and agitated at 500 rpm for 95 hours. The maximum yield of hispidospermidin was reached at around 70 hours of fermentation.

The harvested broth filtrate (17 liters) was adjusted to pH 7 with 1N HCl and applied to a column of Amberlite IRC-50 (Na/H = 7/3) (Rohm and Haas). The column was washed with water and then the active principle was eluted with 0.5N HCl. The eluate was adjusted to pH 7 with 3N NaOH and applied to a column of Diaion HP-21 (Mitsubishi Chemical Industries Ltd.). The active principle was eluted with water and successively 10% aqueous acetone. Combined active fractions were concentrated to remove acetone and extracted ethyl acetate at pH 9. The organic layer was evaporated under reduced pressure to give hispidospermidin (2.6g) as a colorless oil.

The following example illustrates a pharmaceutical preparation containing hispidospermidin provided by the present invention:

Example

Tablets each containing the following ingredients were manufactured in the conventional manner per se:

| | |
|---|---|
| Hispidospermidin | 100 mg |
| Starch | 26 mg |
| Carboxymethylcellulose calcium | 15 mg |
| Crystalline cellulose | 20 mg |
| Magnesium stearate | 4 mg |
| | 165 mg |

**Claims**

**1.** A compound represented by the formula I,

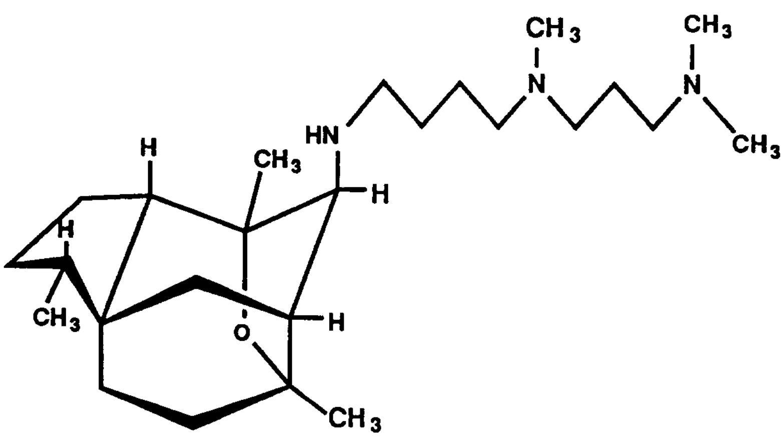

I

and a salt thereof.

**2.** A compound of claim 1 for use as a medicament.

**3.** A process for producing a compound represented by the formula I,

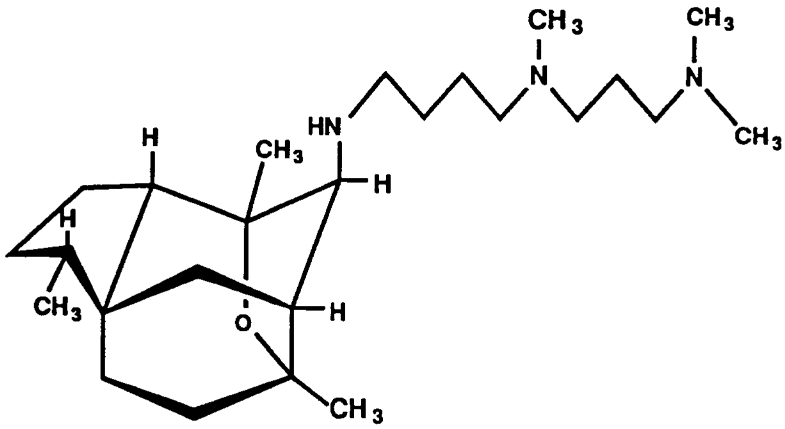

I

which comprises cultivating a microorganism capable of producing the compound represented by the formula I in a culture medium suitable for said microorganism, and isolating the compound represented by the formula I from said culture.

**4.** The process of claim 3 wherein said microorganism belongs to the genus *Chaetosphaeronema*.

**5.** The process of claim 4 wherein said microorganism is *Chaetosphaeronema hispidulum* (CdA) Moesz NR 7127 (FERM BP-4081 and variants thereof.

**6.** The process of claim 3 wherein the cultivating is performed under aerobic conditions.

**7.** The process of claim 6, wherein said culture medium is an aqueous culture medium.

**8.** The process of claim 7 wherein the cultivation is carried out by submerged fermentation.

**9.** A pharmaceutical composition comprising a therapeutically effective amount of a compound represented by the formula I,

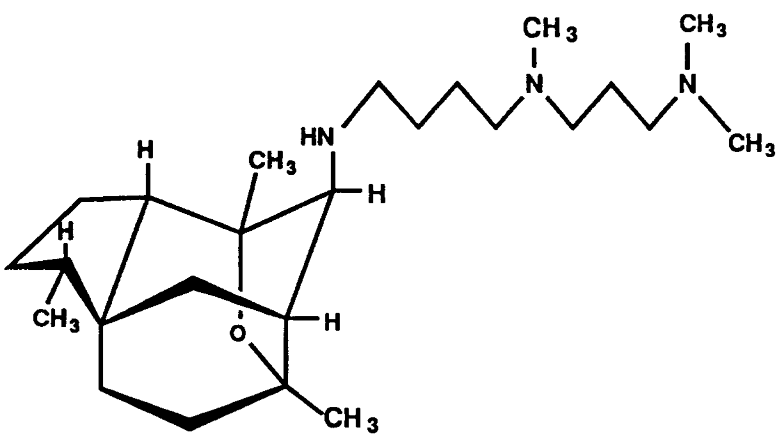

I

or a salt thereof and a therapeutically inert carrier.

**10.** The pharmaceutical composition of claim 9 wherein the amount of said compound represented by the formula I is about 10mg to about 200mg.

**11.** The pharmaceutical composition of claim 9 which is in unit dosage form.

**12.** The pharmaceutical composition of claim 11 wherein said unit dosage form is selected from a group consisting of tablets, coated tablets, dragees, hard gelatine capsules, soft gelatine capsules, solutions, syrups and suspensions.

**13.** The pharmaceutical composition of claim 12 wherein the amount of said compound represented by the formula I in the unit dosage form is from about 10mg to about 200mg.

**14.** The use of a compound of claim 1 in the manufacture of pharmaceutical compositions for the treatment of tumors and inflammatory conditions.

European Patent Office

# EUROPEAN SEARCH REPORT

Application Number
EP 93 11 8790

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 349 224 (UNIVERSITY OF FLORIDA)<br>* claims 1,4,7 *<br>--- | 1,9,14 | C07D307/00<br>C12P17/04<br>A61K31/34 |
| A | JOURNAL OF ORGANIC CHEMISTRY.<br>vol. 49, no. 1 , 1984 , EASTON US<br>pages 133 - 138<br>J. E. NORDLANDER ET AL 'General method for the synthesis of selectively N-alkylated polyamines'<br>* page 135, right column, line 1 - line 5 *<br>----- | 1 | |
| | | | TECHNICAL FIELDS SEARCHED (Int.Cl.5) |
| | | | C07D<br>C12P<br>A61K |

The present search report has been drawn up for all claims

| Place of search | Date of completion of the search | Examiner |
|---|---|---|
| THE HAGUE | 18 February 1994 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)